# EUROPEAN PATENT APPLICATION

(11) **EP 2 036 579 A1**
(43) Date of publication of application: **18.03.2009**
(21) Application number: 08012516.4
(22) Date of filing: 10.07.2008
(51) Int. Cl.: A61L 9/12

(54) **Method for mixing forced airflow with aromatic gas and its relevant mixing apparatus**

(30) Priority: 14.09.2007 CN 200710094084
(71) Applicant: Lee, Jui-Jen, Danshuei Township T'ai pei 251 (TW)
(72) Inventor: Lee, Jui-Jen, Danshuei Township T'ai pei 251 (TW)
(74) Representative: Kador & Partner

(57) **Abstract**

The present invention pertains to a method for mixing forced airflow with aromatic gas and its relevant mixing apparatus. The method mainly mixs the forced airflow with an aromatic gas or other proper gases in time of traveling. That is, passing a certain amount of forced airflow through a flowing route and guiding an aromatic gas to travel along the route so as to ensure the forced airflow can exactly absorb the aromatic gas and obtain the steady consistency after the mixing procedure.

## Description

### BACKGROUND OF THE INVENTION

### 1. Field of the Invention

The present invention relates to a method for mixing gases and the relevant apparatus, more particularly to a method for equably mixing forced airflow or vapor with aromatic gas and its relevant apparatus.

### 2. Description of the Related Art

Generally, the apparatus utilized by incorporating the atomized vapor or gases into some other aromatic gases is usually applied to create a pleasant atmosphere, release the pressure, or cure some other respiratory diseases. One traditional method for the combination is to directly feeding the aromatics into the forced airflow. Another method is to have a container filled with water and pour some aromatics into water(e.g. aroma oil or other appropriative powders); thence to process the admixture of water and aromatics by heating or by supersonic oscillating methods thereby generating water vapor or atomized vapor which has sweet smells; said water vapor or atomized vapor contains the aromatic gases exhaled through the aromatics, and reaches the purposes of producing atmosphere.

By means of the above typical method, the finished water vapor facilely contains an unsteady proportion of the aromatic component with high consistency while directly mixing the water molecule with those aromatics and proceeding it into a vapor state, which may result of the symptom of hypersensitiveness among users who are unable to adjust themselves to those smells. Further, the consistency would be on the decline according to the consuming time and formed of an inconsistent diffusion of the aromatic smelling, thus still requires improvement.

### SUMMARY OF THE INVENTION

Therefore, the object of the present invention is to provide a method for adequately mixing forced airflow with aromatic gas so as to improve the unsteady consistency after the combination.

Another object is to provide an apparatus applied to proceed the mixing procedure which facilitates to generate the aromatic smell via a simple operation.

The method for mixing forced airflow with aromatic gas in conformity with the present invention essentially provides the steps of passing a certain amount of forced airflow through a flowing route and guiding an aromatic gas to travel along the route so as to steadily mix the two materials and generate the admixed gases with sweet smell.

The apparatus with respect to the method of the present invention has a mixing device which is comprised of an upper case, a lower case, and an accommodating room for mounting a gas mixing assembly therein; wherein, the gas mixing assembly mainly includes respective a first opening and a second opening disposed on both sides thereof and defines a flowing route existed inside the room thereby permitting the forced airflow to be smoothly introduced therein and adequately mix with an aromatic gas to produce aromatic smell with steady consistency.

The advantages of the present invention over the known prior arts will become more apparent to those of ordinary skilled in the art by reading the following descriptions with the relating drawings.

### BRIEF DESCRIPTION OF THE DRAWINGS

Fig. 1 is an exploded view showing the preferred embodiment of the present invention;
Fig. 2 is an assembling perspective view of Fig. 1;
Fig. 3 is a cutaway cross-sectional view of Fig. 1;
Fig. 4 is a diagram view showing the passage of the forced airflow and the aromatic gas in time of operation; and
Fig. 5 is a perspective view showing the application of the present invention.

### DETAILED DESCRIPTION OF THE PREFERRED EMBODIMENTS

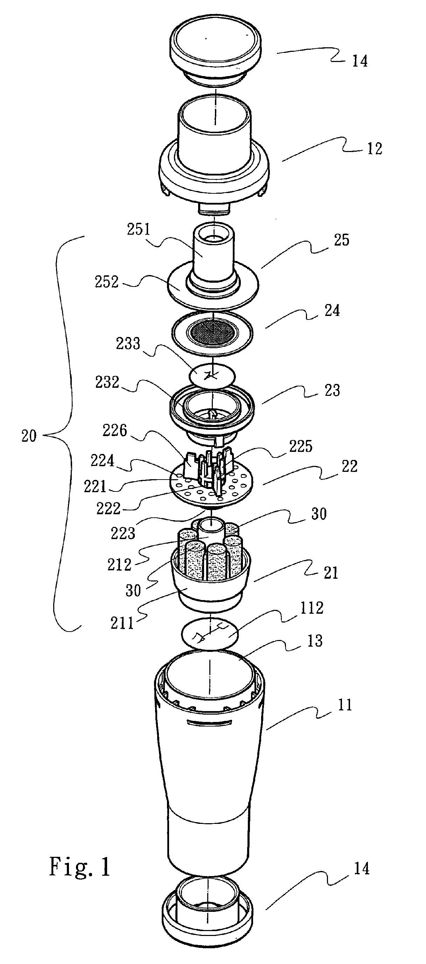

Referring to Fig. **1****,** **3** **and** **4****,** the preferred embodiments of the present invention essentially has a mixing device **10,** which comprises an upper case **12,** a lower case **11,** and an accommodating room **13** defined therebetween for disposing a gas mixing assembly **20** therein and forming a gas mixing chamber **27;** wherein, the mixing device **10** has a first opening **111** and a second opening **121** respectively disposed on both sides thereof so as to form a flowing route functioning as introducing a certain of forced airflow into the accommodating room **13.** Further, a gas mixing assembly **20** is mounted inside the device **10** for mixing the forced airflow with an aromatic gas thereby and assists in diffusing the admixed gas out of the device **10.** The forced airflow herein can be adopted by a high pressure gas or an atomized vapor.

Still further, the first opening **111** is formed at the bottom side of the lower case **11,** and a first anti-counterflow member **112** is disposed on the first opening **111,** and then an extending portion **113** is downwardly extended therefrom for connecting to an external duct **40.** Additionally, the lower case **11** has a room opening on its top end and makes the accommodating room **13** to be formed of a long-tubular contour. The upper case **12** covers the room opening of the lower case **11** and accommodating room 13 communicates with the lower case **11** via the second opening **121** disposed at bottom side of the upper case **12,** and two coverings **14** separately disposed on the upper case **12** and the lower case **11** for blocking the two openings **111** and **121.**

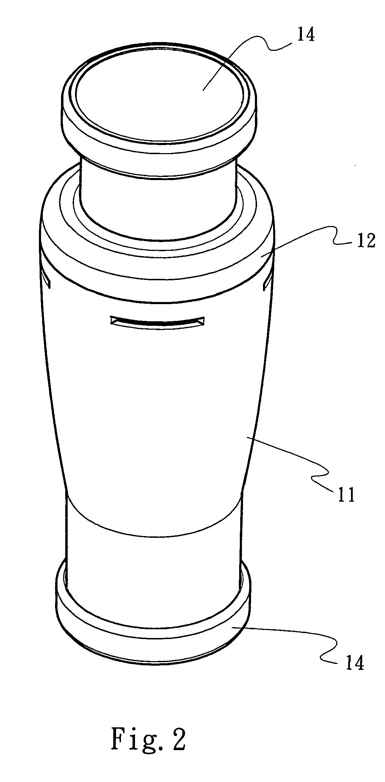

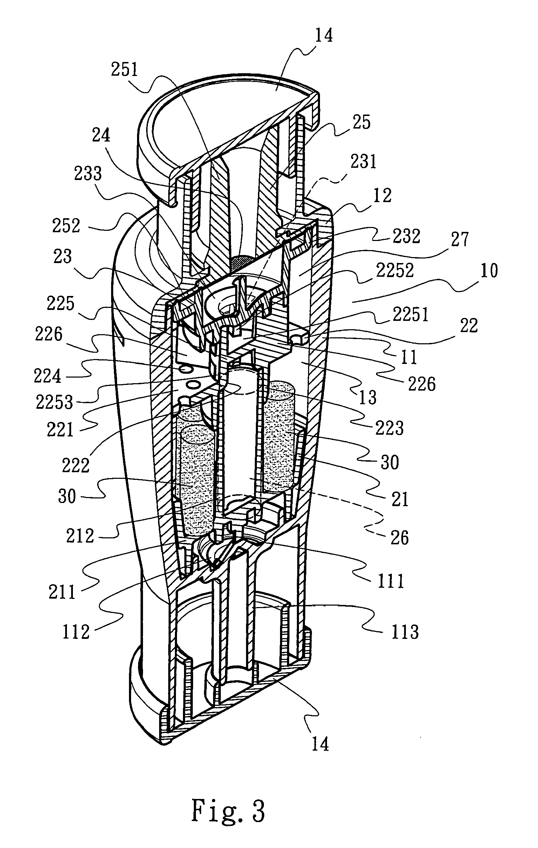

In addition, the gas mixing assembly **20** comprises a containing assembly **21,** a first shield plate **22,** a second shield plate **23,** a filter piece **24,** and a guiding unit **25;** Still referring to Fig 2 and Fig 3, the containing assembly **21** formed of a bowl contour includes a loading case **211** for accurately fitting with the first opening **111,** at least one aroma generator **30** mounted in the loading case **211,** and a conducting tube **212** axially disposed therethrough so that the conducting tube **212** has a bottom end thereof to be in contact with the first opening **111.** Moreover, the aroma generators **30** can contain aroma oil, appropriate powders, or other proper materials. The conducting tube **212** has a filtering unit **26** (e.g. sponge) arranged therein and has an orifice defined at its top end and attached to a first shield plate **22** for communicating with the gas mixing chamber **27.** The first shield plate **22** provides with a clapboard **221** with an outer edge thereof abutting on an inner wall of the accommodating room **13,** a third opening **222** disposed on the clapboard **221,** a connecting portion **223** downwardly extended from the circumference of the third opening **222,** for making the conducting **212** communicated with the third opening **222**, a plurality of apertures **224** disposed on the surface of the clapboard **221,** a fence structure **225** upwardly extends from the third opening **222,** and supporting pieces **226** disposed on the clapboard **221** for engaging with a second shield plate **23;** wherein, the accommodating room **13** is divided into upper and lower chambers and the two chambers communicate with each other via the clapboard **221;** further the upper chamber, namely the gas mixing chamber **27,** is applied for mixing the forced airflow and the aromatic gas. Additionally, the fence structure **225** comprises a shield piece **2251** parallel to the third opening **222** in cross section and a plurality of upper and lower posts **2252, 2253** vertically disposed on an outer circumference of the shield piece **2251;** wherein, the lower posts **2253** extend from an underside of the shield piece **2251** and passes through the third opening **222,** and the upper posts **2252** extend from an upper side of the shield piece **2251.**

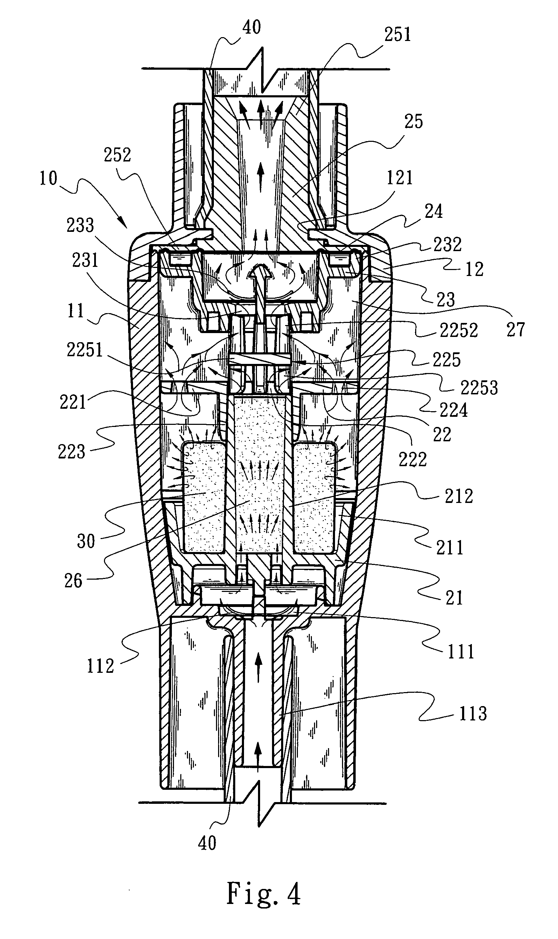

The second shield plate **23** is formed of a quasi-funnel configuration and has a fourth opening **231** defined at its bottom side which is connected to the upper posts **2252** of the fence structure **225,** a shield opening at its top side, and a shoulder portion **232** formed on a periphery of the shield opening; wherein, a second anti-counterflow member **233** is disposed on the fourth opening **231,** and the shoulder portion **232** covers part of the room opening of the lower case **11.** Furthermore, the filter piece **24** is disposed between the second shield plate **23** and the guiding unit **25.** The guiding unit **25** further includes a guiding base **252** attached to the shoulder portion **232** of the second shield plate **23,** and a guiding mouth **251** protruded from the base **252** and going through the second opening **121** to communicate with outsides and connect to the external 40. (refer to Fig. **4****).**

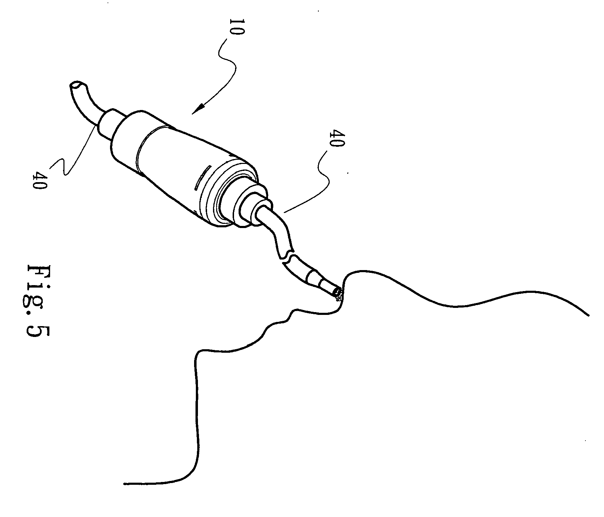

In one operation, the external duct **40** is initially connected to the mixing device **10** as arrowed in Fig. **4** and **5** for feeding forced airflow along a gas feeding entrance of the flowing route into the device **10,** to be more precisely illustrated in Fig. **4****,** the forced airflow is constrainedly introduced from the external 40 to the accommodating room 13 of the mixing device 10, and then into the first opening **111** of the lower case **11,** travels through the conducting tube **212** of the containing assembly 21, and thence passes the third opening **222** to enter into the upper chamber of the clapboard **221** by the assistance of the shield piece **2251.** Simultaneously, in the lower chamber which is located beneath the clapboard **221,** the aroma generators **30** disposed on the containing assembly 21 emit the aromatic gas into the loading case 211, and subsequently the aromatic gas goes across the apertures **224** and diffuses throughout the gas mixing chamber **27** (the upper chamber), so that the forced airflow can completely absorb the aromatic gas therein. Of further operation, the forced airflow can also be directly diffused from the top end of the conducting tube **212** to the upper chamber without passing through the first shield plate **22** for proceeding further combination. Then an admixed gas generated by the combination would go into the gas mixing chamber **27** through the apertures **224.**

Still referring to Fig. **4****,** while the forced airflow and the aromatic gas stay within the gas mixing chamber **27** and mix with each other to form an admixed gas, the new forced airflow incessantly goes into the chamber **27** and expels the admixed gas therefrom. The admixed gas further moves toward the second shield plate **23,** that is, the admixed gas passes across the shield piece 2251 disposed on the fence structure **225,** goes into the second shield plate **23** via the passing the fourth opening **231,** thence enters in sequence to the filter piece **24** and the guiding unit **25,** and finally exits from the guiding mouth **251.** The gas can also be discharged from another external duct **40** which is connected to the upper case **12** (shown in Fig. **5****).** Additionally, the new forced airflow inside the mixing device **10** still keeps absorbing the aromatic gas which has been diffused throughout the chambers, thus obtain the admixed gas with steady proportion of the consistency.

To sum up, the present invention takes advantages of the method for mixing forced airflow with the aromatic gas emitted from the aromatics or powders, so that the forced airflow can absorb the aromatic gas instead of mixing with the substantial materials. Deservedly, users can also arrange suitable introducing devices for introducing other forced airflow (e.g. atomized vapor or other smell gases) into the mixing device **10** through the front side of its gas feeding entrance or the rear side of its gas feeding exit, thereby generating multiple admixed gases. Thus, the present invention facilitates to provide an efficient and facile operation and maintain the steady proportion of the consistency after the combination.

While we have shown and described the embodiment in accordance with the present invention, it should be clear to those skilled in the art that further embodiments may be made without departing from the scope of the present invention.

## Claims

1. A method for mixing forced airflow with aromatic gas comprising steps of passing a certain amount of forced airflow along a flowing route and guiding an aromatic gas to travel along said flowing route so as to mix said forced airflow with said aromatic.

2. The method as claimed in claim 1, wherein said aromatic gas is diffused from at least one aroma generator.

3. The method as claimed in claim 2, wherein said forced airflow travels through an accommodating room where said at least one aroma generator is located.

4. The method as claimed in claim 2, wherein said forced airflow directly travels through a gas mixing chamber without going across an accommodating room where said at least one aroma generator is located; said gas mixing chamber communicates with said accommodating room.

5. The method as claimed in any claims of claim 1 to 4, wherein said forced airflow and said aromatic gas are introduced from an outer environment into said flowing route for proceeding a mixing procedure.

6. The method as claimed in any claims of claim 1 to 5, wherein said forced airflow belongs to an atomized vapor.

7. The method as claimed in any claims of claim 1 to 5, wherein said forced airflow belongs to a high pressure gas.

8. An apparatus for mixing forced airflow with aromatic gas comprising:
a mixing device with an accommodating room defined therein, and said accommodating room receiving a gas mixing assembly disposed therein; wherein, said gas mixing assembly provides with a gas mixing chamber for mixing a certain of forced airflow with an aromatic gas, and said accommodating room defining a flow route therethrough to permit said forced airflow to enter in for the mixture with said aromatic gas and flow out while accomplishing a mixing procedure.

9. The apparatus as claimed in claim 8, wherein at least one aroma generators is disposed within said accommodating room; said accommodating room and said flowing route communicates with said gas mixing chamber, respectively.

10. The apparatus as claimed in claim 8, wherein at least one aroma generator is disposed within said accommodating room; said accommodating room communicates with said gas mixing chamber and said flowing route.

11. The apparatus as claimed in claim 8, 9, or 10, wherein said mixing device has a gas feeding entrance, and a duct member is disposed at the front of said gas feeding entrance for introducing said forced airflow.

12. The apparatus as claimed in claim 8, 9, or 10, wherein said mixing device has a gas feeding exit, and a duct member is disposed behind said gas feeding entrance for introducing said forced airflow.

13. The apparatus as claimed in any one of claims 8 to 12, wherein said mixing device is comprised of an upper case and a lower case.

14. The apparatus as claimed in any one of claims 8 to 12, wherein said mixing device has a first opening and a second opening separately located on both ends thereof so as to communicate with a gas feeding entrance and a gas feeding exit of said flowing route.

15. The apparatus as claimed in claim 14, wherein said gas mixing assembly includes a containing assembly which further has a loading case for allowing said at least one aroma generator mounted therein and a conducting tube axially disposed through said loading case so that said conducting tube has a bottom end thereof to be in contact with said first opening.

16. The apparatus as claimed in claim 15, wherein said conducting tube has a top end thereof to communicate with said gas mixing chamber.

17. The apparatus as claimed in claim 15, wherein said conducting tube has a top end thereof to communicate with said accommodating room where said at least one aroma generator is located.

18. The apparatus as claimed in claim 15, 16 or 17, wherein said conducting tube forms an orifice on a top end thereof and contacts with a first shield plate; said first shield plate provides with a third opening to be in contact with said accommodating room, a clapboard with an outer edge thereof abutting on an inner wall of said accommodating room, and a plurality of apertures disposed on said clapboard, so that said accommodating space is divided into an upper and a lower chambers via said clapboard and said two chambers communicate with each other via said apertures; said upper chamber is applied for mixing said forced airflow and said aromatic gas, and said lower chamber has said loading case disposed therein.

19. The apparatus as claimed in claim 18, wherein a fence structure upwardly extends from said third opening of said first shield plate and further comprises a shield piece parallel to said third opening in cross section and a plurality of upper posts and lower posts vertically disposed on an outer circumference of said shield piece; wherein, said lower posts extend from an underside of said shield piece and passes through said third opening, and said upper posts extend from an upper side of said shield piece.

20. The apparatus as claimed in claim 18 or 19, wherein said first shield plate is attached to a second shield plate.

21. The apparatus as claimed in claim 20, wherein said second shield plate is formed of a quasi-funnel configuration and has a fourth opening defined at its bottom side which is connected to said upper posts of said fence structure, a shield opening at its top side, and a shoulder portion formed on a periphery of said shield opening; said shoulder portion covers part of a room opening defined on a top end of said lower case.

22. The apparatus as claimed in claim 20 or 21, wherein a guiding unit is attached to said second shield plate and includes a guiding mouth protruded therefrom for being attached to said second opening.

23. The apparatus as claimed in claim 22, wherein a filter piece is disposed between said guiding unit and said second shield plate.

24. The apparatus as claimed in claim 15, 16, or 17, wherein said conducting tube has a filtering unit mounted therein.

25. The apparatus as claimed in claim 8, 14, 20, or 22, wherein a first anti-counterflow member is disposed on said gas feeding entrance of said flowing route.

26. The apparatus as claimed in claim 14, 18, 20, 22, or 24, wherein a first anti-counterflow member is disposed on said first opening of said mixing device.

27. The apparatus as claimed in claim 20, 22, 24, or 25, wherein a second anti-counterflow member is disposed on said second shield plate.

28. The apparatus as claimed in claim 27, wherein said second anti-counterflow member is located within the fourth opening defined on said second shield plate.
